# EUROPÄISCHE PATENTSCHRIFT

(11) **EP 3 016 618 B1**
(45) Veröffentlichungstag und Bekanntmachung des Hinweises auf die Patenterteilung: **13.03.2019**
(21) Anmeldenummer: 14736633.0
(22) Anmeldetag: 02.07.2014
(51) Int. Cl.: A61F 2/60, A61F 2/68

(54) **ORTHOPÄDIETECHNISCHE GELENKEINRICHTUNG UND VERFAHREN ZU DEREN STEUERUNG**
ORTHOPAEDIC JOINT AND CONTROL METHOD
PROTHÈSE DE JOINT ET PROCÉDÉ DE CONTRÔLE

(30) Priorität: 03.07.2013 DE 102013011080
(43) Veröffentlichungstag der Anmeldung: 11.05.2016
(73) Patentinhaber: Ottobock SE & Co. KGaA, 37115 Duderstadt (DE)
(72) Erfinder: MOSLER, Lüder, 37115 Duderstadt (DE); PUSCH, Martin, 37115 Duderstadt (DE); PAPPE, Alexander, 37083 Göttingen (DE); WILL, Christian, 37077 Göttingen (DE)
(74) Vertreter: Stornebel, Kai
(86) Internationale Anmeldenummer: PCT/EP2014/001807
(87) Internationale Veröffentlichungsnummer: WO 2015/000588

(56) Entgegenhaltungen:
- US-A1- 2011 264 230

## Beschreibung

Die Erfindung betrifft ein Verfahren zur Steuerung einer orthopädietechnischen Gelenkeinrichtung einer unteren Extremität mit einem Oberteil und einem gelenkig daran gelagerten Unterteil, zwischen denen eine Umwandlungseinrichtung angeordnet ist, über die während einer Verschwenkung des Oberteils relativ zu dem Unterteil mechanische Arbeit aus der Relativbewegung umgewandelt und in zumindest einem Energiespeicher gespeichert und der Gelenkeinrichtung zeitversetzt wieder zugeführt wird, um die Relativbewegung zu unterstützen. Die Erfindung betrifft ebenfalls eine orthopädietechnische Gelenkeinrichtung einer unteren Extremität mit einem Oberteil und einem gelenkig daran gelagerten Unterteil, zwischen denen eine Umwandlungseinrichtung angeordnet ist, über die während einer Verschwenkung des Oberteils relativ zu dem Unterteil mechanische Arbeit aus der Relativbewegung umgewandelt und in zumindest einem Energiespeicher gespeichert und der Gelenkeinrichtung zeitversetzt wieder zugeführt wird, um die Relativbewegung zu unterstützen. Eine solche orthopädietechnische Gelenkeinrichtung ist insbesondere für Prothesen vorteilhaft, kann aber auch bei Orthesen eingesetzt werden.

Die meisten Prothesen der unteren Extremität sind so konstruiert, dass Bewegungsenergie nur in Wärme umgewandelt wird, um das Verhalten der Prothese zu beeinflussen, eine aktive Zufuhr von Energie findet überwiegend nicht statt. Der Prothesennutzer steuert beim Gehen beispielsweise ein Prothesenkniegelenk über seinen Stumpf mit der Hüftmuskulatur. Untersuchungen aus dem Ganglabor haben dabei gezeigt, dass diese Art des Gehens mehr Energie benötigt als das Gehen von gesunden Probanden. Insbesondere beim langsamen Gehen und beim alternierenden Treppaufgehen müssen Prothesennutzer im Vergleich zu gesunden Probanden wesentlich größere Energiemengen aufbringen.

Wird der Prothese oder Orthese in bestimmten Phasen des Gehens Energie zugeführt, muss der Prothesen- oder Orthesennutzer weniger eigene Energie aufbringen, die Bodenfreiheit während der Schwungphase kann erhöht werden und die Bewegung wird insgesamt komfortabler und angenehmer.

Es existieren sogenannte aktive Prothesenkniegelenke, bei denen ein Antrieb den gesamten Bewegungsablauf nachbildet, der Motor ist dabei so ausgelegt, dass die üblichen Gangmuster abgebildet werden können. Dies hat zur Folge, dass der Motor relativ stark ausgelegt werden muss und die Energieversorgung entsprechend schwierig ist. Bei hinreichend starken Motoren kann eine Fehlfunktion in der Steuerung eine Gefährdung des Nutzers darstellen. Wird der Motor schwächer ausgelegt, kann nicht mehr das gesamte Gangspektrum abgedeckt werden. Ein weiterer Nachteil besteht darin, dass nach Einleitung einer bestimmten Bewegung der Nutzer gezwungen wird, der Bewegung zu folgen, selbst wenn sich die Situation oder der Nutzer seine Meinung geändert hat. Der Nutzer hat dann keine Einflussmöglichkeiten mehr auf die Bewegung und wird bewegt.

Die US 2011/264230 A1 betrifft künstliche Gelenke an unteren Extremitäten mit einem Oberteil und einem gelenkig daran angeordneten Unterteil sowie einem Motor zum Aufbringen eines Drehmomentes, zumindest einem passiven elastischen Bauteil sowie einem steuerbaren, variablen Dämpfungselement, um mechanische Energie zu dissipieren. Der Dämpfer kann parallel und/oder in Reihe zu dem elastischen Bauteil oder einer Feder angeordnet sein.

Neben rein motorisch angetriebenen Prothesenkniegelenken ist aus der US 2010/0312363 A1 ein Prothesenkniegelenk mit Motoren und Federspeichern bekannt, bei dem zur Verringerung des Antriebsaufwandes Energie aus der Bewegung in den Federn gespeichert und nach Erreichen einer bestimmten Winkelstellung wieder abgegeben wird. Die Unterstützung kann so dimensioniert sein, dass das Gehen in der Ebene oder auch das Treppensteigen ermöglicht wird.

Aufgabe der vorliegenden Erfindung ist es, ein Verfahren zur Steuerung einer orthopädietechnischen Gelenkeinrichtung und eine solche Gelenkeinrichtung bereitzustellen, mit denen es möglich ist, eine orthopädietechnische Gelenkeinrichtung intuitiv zu nutzen, eine hohe Sicherheit bereitzustellen und eine lange Nutzungsdauer zu ermöglichen.

Erfindungsgemäß wird diese Aufgabe durch ein Verfahren mit den Merkmalen des Hauptanspruches oder eine Vorrichtung mit den Merkmalen des nebengeordneten Anspruchs gelöst. Vorteilhafte Ausgestaltungen und Weiterbildungen der Erfindung sind in den Unteransprüchen, der Beschreibung und den Figuren offenbart.

Das erfindungsgemäße Verfahren zur Steuerung einer orthopädietechnischen Gelenkeinrichtung einer unteren Extremität mit einem Oberteil und einem gelenkig daran gelagerten Unterteil, zwischen denen eine Umwandlungseinrichtung angeordnet ist, über die während einer Verschwenkung des Oberteils relativ zu dem Unterteil mechanische Arbeit aus der Relativbewegung umgewandelt und in zumindest einem Energiespeicher gespeichert und der Gelenkeinrichtung zeitversetzt wieder zugeführt wird, um die Relativbewegung zu unterstützen, sieht vor, dass die gespeicherte Energie rückgewandelt und die Zufuhr mechanischer Arbeit zur und während der Unterstützung der Relativbewegung kontrolliert erfolgt, wobei die Umwandlungseinrichtung und/der Energiespeicher eine Kombination zumindest zweier Federn aufweist, die über verschiedene Winkelbereiche der Gelenkeinrichtung wirksam sind. Bei einer Freigabe von Energie aus einem Energiespeicher, beispielsweise einer Feder, wird die gespeicherte Energie gemäß dem Stand der Technik schlagartig der Gelenkeinrichtung, also dem System aus Oberteil und Unterteil und gelenkiger Lagerung, zugeführt, so dass über einen sehr kurzen Zeitraum eine hohe Energiemenge eingeleitet wird. Erfindungsgemäß ist vorgesehen, dass die gespeicherte Energie kontrolliert dem System wieder zugeführt und in mechanische Arbeit und Unterstützung der Verlagerung von Oberteil zu Unterteil umgewandelt wird, um über einen längeren Zeitraum die Bewegung zu unterstützen, so dass eine an den natürlichen Bewegungsablauf angenäherte Bewegung der prothetischen oder orthetischen Einrichtung erfolgen kann. Eine Anpassung an veränderte Gangmuster, die Geschwindigkeiten oder unterschiedliche Patienten ist gemäß dem Stand der Technik nur außerordentlich aufwendig auszuführen, indem speziell angepasste Federn eingesetzt werden, was für den täglichen Einsatz unpraktikabel ist. Erfindungsgemäß wird dagegen die Energieabgabe in das System kontrolliert, so dass über einen vergleichsweise langen Zeitraum die benötigte Energiemenge eingespeist werden kann, um das Gangbild wie gewünscht zu beeinflussen. Der Zeitpunkt des Ineingrifftretens der Umwandlungseinrichtung zur Veränderung der umzuwandelnden Energiemenge und/oder der zugeführten Energiemenge wird verstellt, so dass beispielsweise in Abhängigkeit von der Gehgeschwindigkeit, der Gehsituation oder den individuellen Parametern des Patienten eingestellt werden kann, wie groß die zu speichernde Energiemenge ist oder wie groß die abzugebende Energiemenge sein muss. Bei einer gewünschten großen Energiemenge ist es vorgesehen, dass ein möglichst früher Eingriff bei der Umwandlung stattfindet, so dass beispielsweise ein Generator sehr früh und sehr lang angetrieben wird oder eine Feder sehr weit vorgespannt wird, um die mechanische Arbeit beim Gehen, beispielsweise beim Fersenauftritt in der Standphasenflexion, maximal in die Lageenergie einer Feder oder elektrische Energie eines Akkumulators oder eines Kondensators umzuwandeln. Wird der Zeitpunkt des Eingriffs bei der Rückwandlung verstellt, beispielsweise durch Verlagerung eines Anschlages oder einer winkelabhängigen Freigabe, wird die Energie zu einem späteren Zeitpunkt des Schrittes eingeleitet, wodurch sich eine Veränderung des Gangbildes erreichen lässt.

Die Zufuhr der mechanischen Arbeit kann dadurch verändert werden, dass dem Energiespeicher extern Energie zugeführt oder entzogen wird. Handelt es sich bei dem Energiespeicher um eine Feder, kann die Zufuhr der Energie dadurch erfolgen, dass die Feder nachgespannt wird, das Entziehen oder das Verringern der Energiemenge kann dadurch erfolgen, dass die Feder entspannt wird, beispielsweise durch Verlagerung eines Federwiderlagers. Ist der Energiespeicher als elektrischer Energiespeicher, beispielsweise Kondensator, Batterie oder Akkumulator ausgebildet, kann die Veränderung der Energiemenge durch Aktivierung eines Generators oder Einleitung aus einem zweiten Energiespeicher erfolgen, die Verringerung der Energiemenge durch Anschließen eines Verbrauchers oder Umleitung in einen zweiten Speicher für elektrische Energie erfolgen.

Eine Weiterbildung der Erfindung sieht vor, dass dem Energiespeicher ein Aktuator zugeordnet ist, über den der Energiespeicher auf ein Mindestniveau befüllt oder gebracht wird, wenn die Relativbewegung dazu nicht ausreicht. Sollte die durch die Bewegung verfügbare Energie nicht ausreichen, um den Energiespeicher für den nächsten Schritt oder den Bewegungsablauf mit ausreichend Energie zu versorgen, wobei die Mindestmenge in Abhängigkeit von der Gehgeschwindigkeit, der Gehsituation und den individuellen Gegebenheiten eines Patienten abhängen, ist erfindungsgemäß vorgesehen, dass während des Gehens und vor der Rückführung von Energie zur Unterstützung der Relativbewegung der Energiespeicher auf ein festgelegtes Niveau aufgefüllt wird, beispielsweise durch Spannen einer Feder oder durch Antreiben eines Generators, der den elektrischen Energiespeicher auflädt.

Um den Zeitpunkt der Bewegungsunterstützung präzise bestimmen zu können ist erfindungsgemäß vorgesehen, dass dem Energiespeicher eine Freigabeeinrichtung zugeordnet ist, über die die Energie aus dem Energiespeicher teilweise oder vollständig freigegeben wird. Die Freigabeeinrichtung bestimmt den Zeitpunkt der Energieabgabe, die Dauer und den Verlauf der Energieabgabe wird bei einer vollständigen Freigabe nicht über die Freigabeeinrichtung gesteuert, sondern über Veränderungen in dem Energiespeicher, also durch Entzug oder Zufuhr von Energie. Bei einer teilweisen Freigabe erfolgte eine Reduzierung der abgegebenen Energiemenge, so dass das Anfangsniveau der Bewegungsunterstützung eingestellt werden kann. Über eine teilweise Freigabe kann eine Anpassung beispielsweise an Gehgeschwindigkeiten, Patienten oder Gehsituationen vorgenommen werden, die Feinbeeinflussung der Unterstützung erfolgt über die Veränderung an dem Energiespeicher.

Die mechanische Arbeit kann in Abhängigkeit von einem Kriterium oder einer Kombination mehrerer Kriterien, nämlich von der Winkelstellung des Oberteils zu dem Unterteil, der Position des Oberteils und/oder des Unterteils im Raum, einer Winkelgeschwindigkeit des Oberteils und/oder des Unterteils und oder einer Relativgeschwindigkeit zwischen dem Oberteil und dem Unterteil und/oder der Belastungssituation und/oder der Beschleunigung des Oberteils und/oder des Unterteils zugeführt werden, so dass eine möglichst genaue zeitliche und mengenmäßige Unterstützung der Bewegung erfolgt. Die Stellungen der Oberteile und Unterteile zueinander und im Raum können durch Winkelsensoren oder Inertialsensoren bestimmt werden, die Geschwindigkeiten zueinander oder innerhalb des Raumes oder die Beschleunigungen durch Beschleunigungssensoren oder einer Kombination aus Winkelsensor und Beschleunigungssensor und die Belastungssituationen können über Kraftsensoren ermittelt werden. Über die Sensoren lässt sich nicht nur der Zeitpunkt der Freigabe der Energie, sondern auch die jeweilige Gangsituation, die Gehgeschwindigkeit sowie die aktuelle Position der jeweiligen Komponenten zueinander oder im Raum bestimmen, worüber es ermöglicht wird, die Menge und den Verlauf der Energiezufuhr zur Unterstützung der Bewegung zu bestimmen und zu steuern.

Eine Weiterbildung der Erfindung sieht vor, dass die Energie aus dem Energiespeicher in Abhängigkeit von einem Kriterium oder einer Kombination mehrerer Kriterien, nämlich von der Winkelstellung des Oberteils zu dem Unterteil, der Position des Oberteils und/oder des Unterteils im Raum, eine Winkelgeschwindigkeit des Oberteils und/oder des Unterteils und/oder der Relativgeschwindigkeit zwischen dem Oberteil und dem Unterteil und/oder der Belastungssituation und/oder der Beschleunigung des Oberteils und/oder des Unterteils entzogen oder diesem wieder zugeführt wird, um die kontrollierte Steuerung der Bewegung zu ermöglichen.

Der Energiespeicher kann von einem Aktuator aufgeladen werden, wenn die Umwandlungseinrichtung nicht aufgrund der Relativbewegung zwischen dem Oberteil und dem Unterteil aktiv ist, so dass der Aktuator nicht gegen die Relativbewegung arbeiten muss. Darüber hinaus hat die zeitliche Einordnung der Aufladung des Energiespeichers durch den Aktuator in eine Phase, in der keine mechanische Arbeit aus der Gelenkeinrichtung umgewandelt wird, den Vorteil, dass über einen langen Zeitraum Energie eingespeichert werden kann, was dazu führt, dass der Aktuator entsprechend klein dimensioniert werden kann, um über einen großem Zeitraum die gewünschte Menge bereitstellen zu können. Wird beispielsweise eine Feder über einen Motor als Aktuator gespannt, kann dieser klein ausgelegt werden und mit einer Übersetzung mit der Feder gekoppelt werden, so dass über einen vergleichsweise großen Zeitraum die Feder gespannt werden kann. Gleiches gilt für die Umwandlung und Speicherung elektrischer Energie.

Eine Weiterbildung der Erfindung sieht vor, dass die Relativbewegung zusätzlich zu der Beeinflussung des Energiespeichers über eine Dämpfereinrichtung beeinflusst wird, so dass nicht ausschließlich über den Energiespeicher die Steuerung erfolgen muss, was dazu führt, dass eine große Variationsmöglichkeit bei der Beeinflussung des Gangbildes vorliegt. Darüber hinaus können Belastungsspitzen über eine zusätzliche Dämpfereinrichtung leichter aufgefangen werden.

Die orthopädietechnische Gelenkeinrichtung einer unteren Extremität mit einem Oberteil und einem gelenkig daran gelagerten Unterteil, zwischen denen eine Umwandlungseinrichtung angeordnet ist, über die während einer Verschwenkung des Oberteils relativ zu dem Unterteil mechanische Arbeit aus der Relativbewegung umgewandelt und in zumindest einen Energiespeicher gespeichert und der Gelenkeinrichtung zeitversetzt wieder zugeführt wird, um die Relativbewegung zu unterstützen sieht vor, dass dem Energiespeicher ein Aktuator zugeordnet ist, der während der Unterstützung der Relativbewegung dem Energiespeicher kontrolliert Energie zuführt oder entzieht. Durch die kontrollierte Zufuhr oder den kontrollierten Entzug von Energie aus dem Energiespeicher über einen Aktuator ist eine besonders einfache und zuverlässige Beeinflussung des Gangbildes bei semiaktiven Gelenkeinrichtungen, insbesondere bei semiaktiven Prothesenkniegelenken möglich. Die Umwandlungseinrichtung, der Energiespeicher oder beide Komponenten weist oder weisen eine Kombination zumindest zweier Federn auf, die über verschiedene Winkelbereiche der Gelenkeinrichtung wirksam sind. Die Umwandlungseinrichtung istverstellbar mit dem Oberteil und/oder mit dem Unterteil gekoppelt, um eine Eingriffsposition oder einen Verstellweg zu verlagern. Dadurch ist es möglich, sowohl die Energiemenge als auch den Zeitpunkt der Energiezufuhr wie gewünscht zu beeinflussen.

Die Umwandlungseinrichtung kann als Feder oder Wandler, beispielsweise Generator ausgebildet sein, um die mechanische Arbeit, die bei einer Relativbewegung zwischen dem Oberteil und dem Unterteil anfällt, entweder in Lageenergie in einer Feder oder elektrische Energie in einer elektrischen Speichereinrichtung, beispielsweise in Gestalt eines Akkumulators, in einer aufladbaren Batterie oder in einem Kondensator oder in einer anderen Energieform zu speichern.

Der Energiespeicher kann als Feder, Federspeicher, beispielsweise Druckspeicher in Verbindung mit Fluidaktuatoren, oder Akkumulator ausgebildet sein, wobei unter einem Akkumulator auch ein Kondensator oder eine aufladbare Batterie verstanden wird.

Eine separate Dämpfereinrichtung kann zwischen dem Oberteil und dem Unterteil angeordnet sein, um die Relativbewegung bei Unterstützung durch den Energiespeicher besser kontrollieren zu können. Durch die Überlagerung der Beeinflussung des Energiespeichers mit der separaten Dämpfereinrichtung kann eine präzisere und sichere Beeinflussung des Gangbildes erfolgen.

Die separate Dämpfereinrichtung ist verstellbar ausgebildet, um in Abhängigkeit von Sensordaten, beispielsweise bezüglich des Gelenkwinkels, der Ganggeschwindigkeit, einer Winkelgeschwindigkeit oder eines Absolutwinkels eines Oberteils und/oder eines Oberteils eine angepasste Dämpfung bereitzustellen. Die Dämpfereinrichtung kann über einen Aktuator verstellt werden, um eine Verringerung oder Vergrößerung der Dämpfung zu erreichen.

Die Umwandlungseinrichtung, der Energiespeicher oder beide Komponenten kann oder können als eine Kombination zumindest zweier Federn aufweisen, die über verschiedene Winkelbereiche der Gelenkeinrichtung wirksam sind. Die Federn können unterschiedliche Federsteifigkeiten aufweisen, so dass eine an die Erfordernisse der jeweiligen Bewegung angepasste Kraftzufuhr ermöglicht wird. Ist beispielsweise vorgesehen, dass über die Umwandlungseinrichtung eine Schwungphaseneinleitung eines Kniegelenks unterstützt oder eingeleitet werden soll, ist zu berücksichtigen, dass ein hierfür erforderliches Unterstützungsmoment nicht linear ist. Ein nichtlineares Unterstützungsmoment kann mit einer linearen Feder erzeugt werden, wenn diese über einen Dämpfer und/oder einen Aktor in ihrem Verhalten beeinflusst wird. Um den hierfür notwendigen Aufwand zu vermeiden, können zwei oder mehr Federn miteinander gekoppelt oder verschaltet werden, wobei die Federn unterschiedliche Federcharakteristiken oder Federsteifigkeiten aufweisen können und über verschiedene Winkelbereiche wirksam sind. Wird beispielsweise zu Beginn der Einbeugung ein hohes Unterstützungsmoment gewünscht, kann durch eine parallel oder seriell geschaltete Federkombination dies einfach erreicht werden. Die Winkelabhängigkeit kann durch eine Längsführung einer der Federn erreicht werden, die z.B. ab einer vollständigen Entspannung in einem Langloch oder teleskopartig geführt ist, so dass keine Einwirkung dieser Feder auf die Bewegung erfolgt. Eine zweite Feder oder weitere Federn, beispielsweise mit einer anderen Steifigkeit oder Federlänge, wirkt über einen längeren Zeitraum oder einen größeren Verschwenkwinkel der Gelenkeinrichtung. Bei einem parallelen Zusammenschalten ergibt sich ein nichtlinearer Verlauf, der an den gewünschten Momentenverlauf einfach angepasst werden kann. Vorteilhafterweise ist der Einsatz eines Dämpfers nicht zur Anpassung des Momentenverlaufes notwendig, so dass weniger Energie in nicht nutzbare Wärme umgewandelt wird. Wäre nur eine Feder vorhanden, müsste diese stärker ausgelegt und die in gewissen Phasen der Beugebewegung überschüssige Energie weggedämpft werden. Darüber hinaus wird kein Aufwand wird eine Dämpferregelung benötigt.

Ausführungsbeispiele der Erfindung werden anhand der nachfolgenden Ausführungsbeispiele näher erläutert. Gleiche Bezugszeichen bezeichnen gleiche Komponenten. Es zeigen:
- Figur 1: eine Gelenkeinrichtung mit einer elastischen Sehne als Energiespeicher;
- Figur 2: eine Variante der Figur 1 mit einer verschieblichen Federanbindung;
- Figur 3: eine Variante mit einer im Unterteil angeordneten Feder;
- Figur 4: eine Variante der Figur 3 mit einem zwischengeschalteten Aktuator;
- Figur 5: eine Variante mit einem Drillfaden als Energiespeicher;
- Figur 6: eine Darstellung von Antriebsmomentverläufen bei unterschiedlichen Gehgeschwindigkeiten;
- Figur 7: eine Darstellung unterschiedlicher Verlagerungswege über einen Gelenkwinkel bei verschiedenen Gehgeschwindigkeiten;
- Figur 8: eine Darstellung eines Hebelarmverlaufes über einen Gelenkwinkel;
- Figur 9: eine Darstellung einer Flexionsdämpfungseinstellung eines Dämpfers über einen Kniewinkel; sowie
- Figur 10: eine Darstellung eines Momentenverlaufes bei parallel geschalteten Federn.

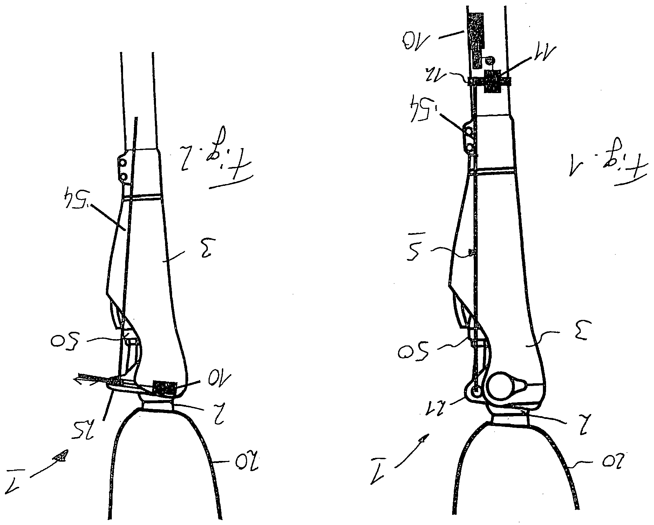

Figur 1 zeigt eine orthopädietechnische Gelenkeinrichtung in Gestalt eines Prothesenkniegelenkes mit einem Oberteil 2, an dem ein Oberschenkelschaft 20 zur Aufnahme eines Oberschenkelstumpfes angeordnet ist. Distal zu dem Oberteil 2 ist ein Unterteil 3 gelenkig befestigt, so dass das Oberteil 2 relativ zu einem Unterteil 3 verschwenkt werden kann. An dem Oberteil 2 ist rückwärtig ein Ausleger 21 ausgebildet, an dem einerseits eine Dämpfereinrichtung 50 in Gestalt eines hydraulischen oder pneumatischen Dämpfers und andererseits ein Energiespeicher 54 in Gestalt einer elastischen Sehne angeordnet ist. Die elastische Sehne ist über ein Übersetzungsgetriebe 11 mit einem Aktuator 10 in Gestalt eines Elektromotors verbunden. Der Elektromotor ist in einem Unterschenkelrohr angeordnet, das an dem Unterteil 3 befestigt ist. Der Energiespeicher 5 in Gestalt der elastischen Sehne ist an dem Übersetzungsgetriebe 11 und einem Ausleger 12 befestigt, wird der Motor 10 aktiviert, wirkt dieser über das Übersetzungsgetriebe 11 auf den Ausleger 12 ein und kann die elastische Sehne 54 entweder spannen oder entspannen, indem der Ausleger 12 in distale oder proximale Richtung verlagert oder in die eine oder andere Richtung verdreht wird, um die elastische Sehne auf- oder abzurollen. Der Ausleger 12 bildet somit einen verlagerbaren Lagerungspunkt des Energiespeichers 5, wodurch es möglich ist, bei einer Extensionsbewegung des Unterteils 3 den Beginn eines Spannvorganges der elastischen Sehne 54 einzustellen. Über den Ausleger 12 kann ein verlagerbarer, elastischer Streckanschlag realisiert werden, der über den Aktuator 10 verstellt wird. Über eine Extensionsbewegung des Unterteils 10 wird der als Feder ausgebildete Energiespeicher 54 gespannt und nimmt einen Teil der Bewegungsenergie des Unterteils 3 auf. Dies kann beispielsweise am Ende der Schwungphase oder nach dem Fersenstoß und der Standphasenflexion erfolgen. Im Rahmen der Standphasenextension wird die Feder 54 gespannt und während der Standphase weiter gespannt beibehalten.

In der terminalen Standphase kann zur Unterstützung der Schwungphaseneinleitung die gespeicherte Energie wieder abgegeben werden, die elastische Sehne 54 zieht sich zusammen und wandelt die Lageenergie in mechanische Arbeit um, um die Flexion des Unterteils 3 zu unterstützen. Soll mehr Energie in dem Energiespeicher 54 gespeichert werden, spannt der Aktuator 10 die elastische Sehne 54 vor, indem der Ausleger 12 distal verlagert oder in Aufrollrichtung verdreht wird, soll weniger Energie gespeichert werden, wird der Ausleger 12 proximal verlagert oder die Sehne abgerollt. Im dargestellten Ausführungsbeispiel ist die Energiespeichereinrichtung 54 gleichzeitig die Umwandlungseinrichtung 5, in der die mechanische Arbeit aus der Relativbewegung in potenzielle Energie umgewandelt wird.

Zusätzlich zu der Unwandlungseinrichtung 5 bzw. dem Energiespeicher 54 ist ein separater Dämpfer 50 in Gestalt eines Hydraulik- oder Pneumatikdämpfers vorgesehen, der verstellbar ausgebildet ist, so dass über die Dämpfereinrichtung 50 die Dämpfung während des Gehens sowohl in Flexionsrichtung als auch Extensionsrichtung beeinflusst werden kann.

Zur kontrollierten Unterstützung bei der Schwungphaseneinleitung ist es vorgesehen, dass über den Aktuator 10, die Übersetzung 11 und die Verlagerung oder Verdrehung des Auslegers 12 eine Veränderung der Vorspannung der elastischen Sehne 54 erfolgt, um die Energieabgabe besser kontrollieren zu können. Es hat sich herausgestellt, dass eine Feder alleine als Kraftspeicher eine zu große und zu schnelle Krafteinleitung bewirkt, was von den Patienten als unangenehm empfunden werden kann. Um sowohl den Zeitraum der Energieeinleitung als auch die Energiemenge und die Leistung kontrollieren zu können, kann in Abhängigkeit von der Winkelstellung des Oberteils 2 zum Unterteil 3, der Winkelstellung des Oberteils 2 und/oder des Unterteils 3 zueinander oder im Raum, den Winkelgeschwindigkeiten oder der Ganggeschwindigkeit eine Manipulation am Energiespeicher 54 vorgenommen werden, um die Leistung zu begrenzen und darüber hinaus den zeitlichen Ablauf der Energieabgabe zu steuern. Durch Entspannen der Feder 54 ist es möglich, weniger Energie in die Gelenkeinrichtung 1 einzubringen, durch Nachspannen der Feder 54 ist es möglich, eine Unterstützung der Flexion über einen längeren Zeitraum hinweg und über einen größeren Flexionswinkel hinweg aufrechtzuerhalten, um das gewünschte harmonische Gangbild zu erreichen.

Eine Variante der Erfindung ist in der Figur 2 dargestellt, bei der statt der Entspannung oder Spannung der Feder 54 im Wesentlichen in ihrer Längserstreckung eine Verlagerung an dem distalen Lagerungspunkt erfolgt. Der obere Befestigungspunkt ist in einer verschieblichen Federanbindung 25 geführt, die über den Aktuator 10 in Richtung des Doppelpfeils hin- und herschiebbar ist. Je nach Anlenkpunkt und Bewegungsrichtung wird die elastische Sehne 54 gespannt oder entspannt. Sowohl in der Figur 1 als auch in der Figur 2 wird die Energie aus der Extensionsbewegung in der elastischen Sehne 54 gespeichert. Nach Beendigung der Extensionsbewegung ist es möglich, dass der Motor 10 die Sehne 54 anschließend nachspannen kann, wenn die erwartete aufzubringende Energie nicht ausreicht, um eine gewünschte Unterstützung bei der Flexionseinleitung zu bewirken. Das Nachspannen erfolgt vorteilhafterweise dann, wenn sich die Gelenkeinrichtung 1 in einem vollständig extendierten Zustand befindet, um möglichst wenig gegen eine Vorspannungbewegung anarbeiten zu müssen. Über die Verstellung entweder der Vorspannung der elastischen Sehne 54 oder der proximalen Lagerungsposition ist es möglich, den Streckwinkel einzustellen, ab dem die Umwandlungseinrichtung 5 aktiv wird, worüber sich auch einstellen lässt, wie viel Energie in dem Energiespeicher 5 gespeichert werden soll.

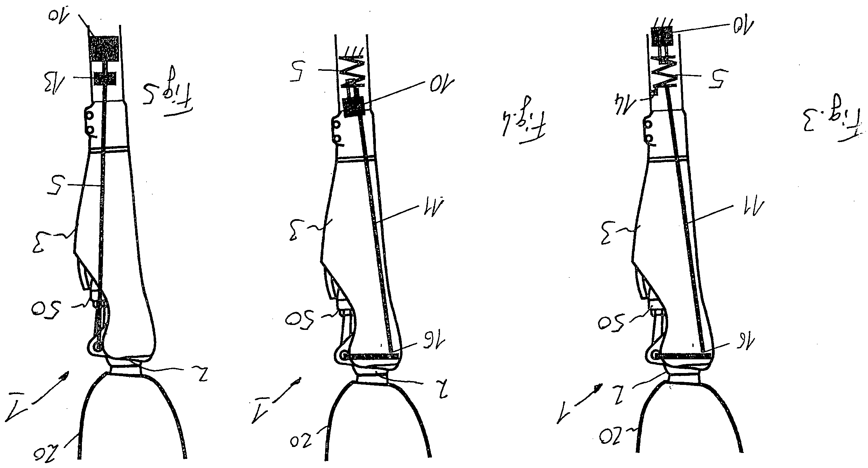

In der Figur 3 ist eine Variante der Gelenkeinrichtung 1 dargestellt, bei der die Umwandlungseinrichtung 5 in Gestalt der Feder 54 im Unterschenkelrohr angeordnet ist. Der Aktuator 10 ist mit der Feder 5 verbunden und kann diese entweder komprimieren oder aufwickeln, je nach Ausgestaltung der Feder als Druckfeder oder Spiralfeder. Die Feder 5 ist über eine Druckstange 11 mit einem Anschlag 16 gekoppelt, der an dem Oberteil 2 befestigt ist. Durch eine Aktivierung des Motors 10 kann der Fußpunkt der Feder 5 verändert werden, wodurch sich über die Druckstange 11 einstellen lässt, wann die Feder 5 mit dem Anschlag 16 in Kontakt kommt. Je früher die Druckstange 11 mit dem Anschlag 16 in Kontakt kommt, desto größer ist der Verstellweg und die Kompression der Feder 5, so dass entsprechend mehr Energie in der Feder 5 gespeichert wird. Dementsprechend wird bei einer Rückumwandlung mehr Energie aus der Feder über die Druckstange 11 auf den Anschlag 16 übertragen, so dass eine vergrößerte Flexionsunterstützung erreicht werden kann. Um die Energieabgabe zu beeinflussen, wird die Feder 5 bei der Bewegungsunterstützung entweder gespannt oder entspannt.

Eine Variante ist in der Figur 4 dargestellt, die im Wesentlichen der Figur 3 entspricht, jedoch ist der Aktuator 10 gegebenenfalls mit Spindeltrieb und einem Freilauf in eine Richtung zwischen der Feder 5 und der Druckstange 10 angeordnet, so dass der Fußpunkt der Feder 5 fest bleibt, sich jedoch die Feder 5 mit dem Motor 10 vorspannen lässt. Wird eine Flexionsunterstützung eingeleitet, muss der Motor 10 mitdrehen, um die Energie freizugeben und über die Druckstange 11 und den Anschlag 16 auf die Gelenkeinrichtung zu übertragen, wodurch sich eine besonders gute Kontrolle über die Energiefreigabe erreichen lässt. Ebenso ist es möglich, die Energiefreigabe zu stoppen, was sinnvoll sein kann, wenn eine Situation falsch eingeschätzt wurde.

In der Figur 5 ist ein Drillfaden als Energiespeicher 54 und Umwandlungseinrichtung 5 gezeigt, bei der über eine Verdrillung von Fäden eine Verkürzung erreicht wird. Durch Zunahme oder Abnahme der Verdrillung kann der Kontaktpunkt eingestellt werden, ab dem der Drillfaden Zugkräfte aufbaut. Zwischen dem Motor 10 und dem Drillfaden 54 ist eine Axialentkopplung 13 vorgesehen.

Neben der dargestellten Ausführungsform des Energiespeichers als Feder kann dieser auch gegebenenfalls über ein Übersetzungsgetriebe und einen Generator als elektrischer Energiespeicher in Gestalt einer Batterie, eines Akkumulators oder eines Kondensators ausgebildet sein. Zur Rückumwandlung der gespeicherten elektrischen Energie wird der Generator als Motor geschaltet, so dass ein Antrieb und eine Unterstützung der Relativverlagerung des Unterteils 3 zu dem Oberteil 2 erfolgen können. Zur Erhöhung der Energiemenge kann ein Generator dem elektrischen Energiespeicher zugeordnet sein, ebenso ist es möglich, einen weiteren Energiespeicher vorzusehen, der als Puffer dient, in den überschüssige elektrische Energie eingespeichert oder aus dem zusätzlich benötigte Energie bereitgestellt wird.

Die Federn als Energiespeicher 54 können als Zugfedern, Druckfedern, Drehfedern oder Elastomerelemente ausgebildet sein, die ab einem bestimmten Streckwinkel, der von dem Aktuator 10 eingestellt wird, in Kontakt kommen und sowohl mechanische Arbeit in Energie ab diesem Zeitpunkt umwandeln als auch wieder zur Bewegungsunterstützung zurückführen. Die Feder nimmt dabei die Energie aus der Bewegung in Extensionsrichtung auf, und dient gleichzeitig als Bremseinrichtung und Extensionsanschlag. Bei der Schwungphaseneinleitung wird die Energie wieder abgegeben und hilft dem Nutzer, die Schwungphase einzuleiten. Über den Aktuator 10 kann der Zeitpunkt des Kontaktes der Feder bei der Energieabgabe verstellt werden, so dass unterschiedliche, kontrollierte Unterstützungen bei verschiedenen Gehgeschwindigkeiten möglich sind. Ebenso ist es möglich, dass über den Motor 10 die jeweilige Feder nachgespannt wird, falls die durch die vorhergehende Bewegung gespeicherter Energie nicht ausreicht, um eine ausreichende Unterstützung bereitzustellen, beispielsweise kann beim besonders langsamen Gehen oder beim Treppabgehen die mechanische Arbeit nicht ausreichen, um die Feder ausreichend zu spannen. Der Feder 5 kann, wie in der Figur 3 gezeigt, eine Freigabeeinrichtung 14 zugeordnet sein, über die der Auslösezeitpunkt zur Freigabe der gespeicherten Energie zusätzlich abgesichert werden kann.

Um das Auslösen der Freigabe abzusichern, kann die Gelenkeinrichtung 1 eine Sicherung enthalten, die durch die Hydraulik in dem Dämpfer 50 oder durch eine Steuerung des Motors 10 ausgebildet wird, die sicherstellen, dass die aufgebrachte Federenergie rechtzeitig wieder abgebaut wird.

Aufgrund der Tatsache, dass die Bewegungsenergie bei der Extension zumindest teilweise gespeichert wird, kann die motorische Unterstützung sehr sparsam arbeiten. Die Batterie für den Aktuator 10 kann klein und leicht ausfallen, ebenso wie der Aktuator 10 selbst, da der Aktuator 10 beim Nachspannen in der Standphase ausreichend Zeit hat, die Feder zu spannen und das Einspeisen der Energie nicht so schnell erfolgen muss, wie es die Abgabe für die Schwungphaseneinleitung erfordert. Der Motor 10 steuert die Energiefreigabe aus der Feder, gegebenenfalls in Verbindung mit den separaten Dämpfer 20. Die Flexionsunterstützung durch den Energiespeicher hilft beim Erreichen des notwendigen Beugewinkels beim alternierenden Treppensteigen sowie beim Übersteigen von Hindernissen und erspart Hüftarbeit.

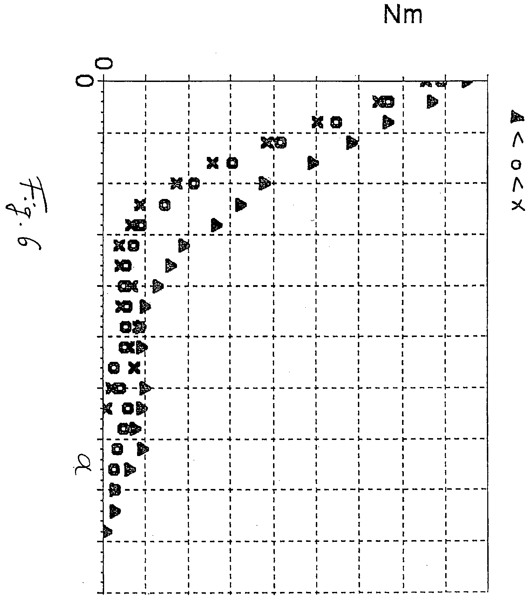

In der Figur 6 ist beispielhaft das Antriebsmoment bei verschiedenen Gehgeschwindigkeiten über den Gelenkwinkel α dargestellt. Die Darstellung erfolgt für drei unterschiedliche Gehgeschwindigkeiten, wobei die jeweiligen Gehgeschwindigkeiten durch unterschiedliche Symbole in dem Diagramm dargestellt werden, die niedrigste Gehgeschwindigkeit ist mit einem Dreieck gekennzeichnet, die mittlere durch einen Kreis und die höchste Gehgeschwindigkeit durch ein X. Das Antriebsmoment ist das effektive Antriebsmoment in Nm, also die durch die Speichereinrichtung 5 gespeicherte und wieder zugeführte Energie abzüglich der Verluste wie Dämpfung oder Reibung. Es ist zu erkennen, dass anfänglich mit einem sehr hohen Antriebsmoment gearbeitet wird, um die Flexionsunterstützung zu Beginn bereitstellen zu können. Bei zunehmendem Gelenkwinkel a, vorliegend dem Kniewinkel, der von einer maximalen Extensionsstellung aus gemessen wird, fällt das aufzubringende Antriebsmoment zunächst steil ab, bleibt über einen kleinen Winkelbereich konstant, steigt kurz wieder an und fällt dann bis zur maximalen Flexion auf null ab. Es ist zu erkennen, dass die Flexionsunterstützung bei geringen Gehgeschwindigkeiten, repräsentiert durch das Dreieck, größer ausfällt als bei hohen Gehgeschwindigkeiten. Der Antriebsmomentverlauf, wie er in der Figur 6 zeigt ist, ist mit einer Feder ohne einen motorischen Einfluss durch Nachspannen oder Entspannen nicht erzeugbar, da erfindungsgemäß nach dem starken Rückgang des Antriebsmomentes über einen weiteren Zeitraum das Moment bis zum Erreichen des Maximalwinkels aufrechterhalten wird.

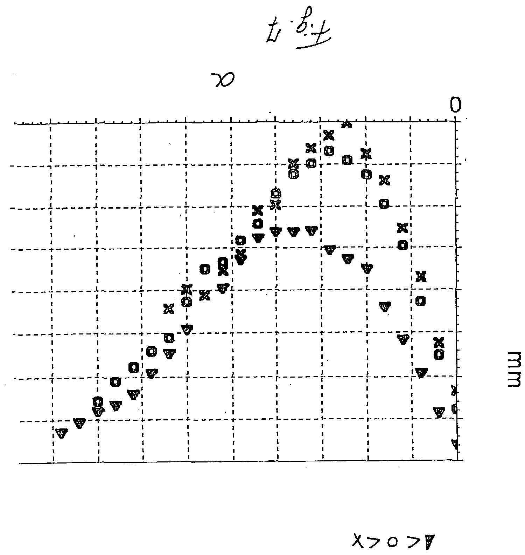

Figur 7 zeigt ein Diagramm, bei dem der Zugweg des Motors 10 über den Gelenkwinkel α aufgetragen ist. Es sind verschiedene Gehgeschwindigkeiten aufgeführt, die wiederum durch ein Dreieck, einen Kreis und ein X gekennzeichnet sind, die geringste Gehgeschwindigkeit ist durch ein Dreieck repräsentiert. Die Darstellung bezieht sich auf die translatorische Bewegung des Motors 10 bei den Ausführungsformen der Figuren 1, 3 und 5. Der Verlagerungsweg gemäß Figur 7 ist so abgestimmt, dass die Antriebsmomentkurve gemäß Figur 6 erreicht werden kann. Der jeweilige Verlauf ist für jede gewählte Feder verschieden und kann je nach Eigenschaft der Feder zu einem größeren oder kleineren Verlagerungsweg führen. Ziel ist es, eine möglichst geringe Verlagerung und damit Spannung der Feder zu erreichen. Beim Start der Beugeeinleitung ist zu erkennen, dass der Motor die Feder zurückgleiten lässt, um eine möglichst schnelle Kraftreduzierung zu erreichen, damit das Gefühl einer kontrollierten Flexion weiter aufrechterhalten bleibt. Bei größeren Winkeln wird die Feder wieder gespannt, um die Kraft aufrechtzuerhalten bzw. wieder zu erhöhen. Auch hier ist es signifikant, dass bei langsamen Gehgeschwindigkeiten eine vergrößerte Unterstützung notwendig ist. Die Freigabe der Feder und damit der Energie zur Flexionsunterstützung und der Antrieb des Motors erfolgen gleichzeitig, so dass über den gesamten Verlauf der Flexionsunterstützung eine Kontrolle möglich ist.

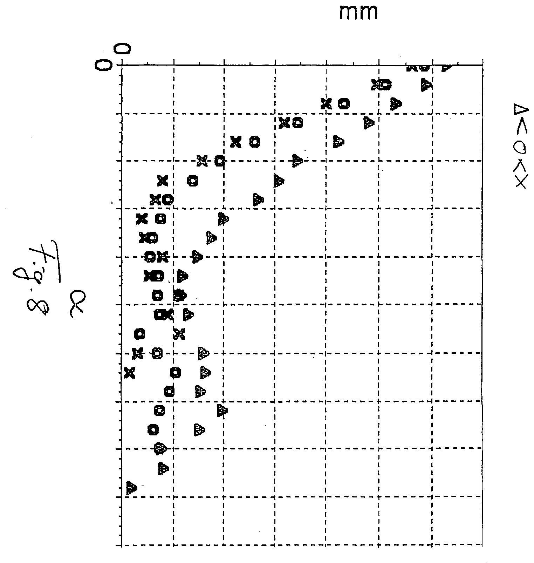

Die Figur 8 zeigt die Veränderung des Hebelarms gemäß einer Ausführungsform der Figur 2 über den Gelenkwinkel α für unterschiedliche Gehgeschwindigkeiten. Auch hier wird gleichzeitig mit der Federfreigabe der Motor 10 aktiviert, um den Hebelarm zu verstellen. Zunächst wird der Hebelarm schnell verkleinert, um einen Kraftabbau zu bewirken, anschließend wird der Hebelarm wieder vergrößert, um über einen größeren Winkelbereich α Kraft aufzubringen und die Flexionsbewegung zu unterstützen.

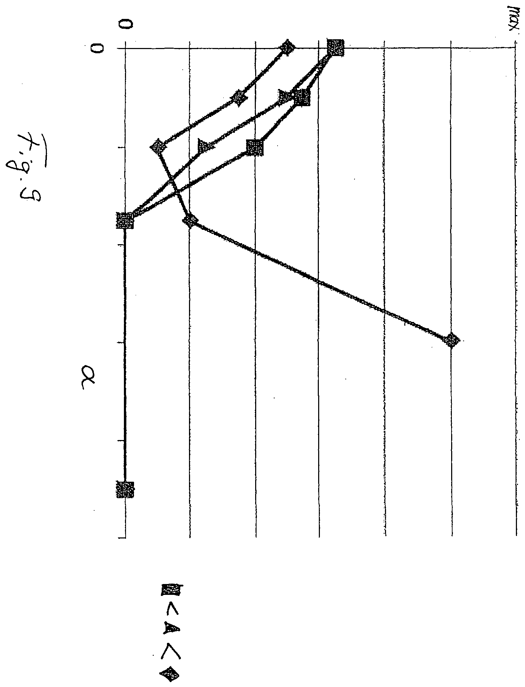

In der Figur 9 ist die Flexionsdämpfungseinstellung der Dämpfereinrichtung 50 über den Gelenkwinkel α bei verschiedenen Geschwindigkeiten dargestellt. Abweichend zu den vorherigen Figuren ist die niedrigste Gehgeschwindigkeit durch ein Quadrat gekennzeichnet, die mittlere durch ein Dreieck und die höchste Gehgeschwindigkeit durch eine Raute. Zunächst ist eine mittlere Flexionsdämpfungseinstellung der Dämpfereinheit 50 vorgesehen, die mit zunehmendem Gelenkwinkel α abnimmt. Bei hohen Gehgeschwindigkeiten kann zum Ende der Schwungphase eine Anhebung der Flexionsdämpfung erfolgen, um ein übermäßiges Einbeugen des Unterteils 3 zu vermeiden. Durch die Veränderung der Flexionsdämpfungseinstellung in der Dämpfereinheit 50 ist es möglich, in Verbindung mit der Motorsteuerung eine effektive und sichere sowie einfache Steuerung der Energieeinbringung zur Bewegungsunterstützung durchzuführen.

Neben der dargestellten Ausführungsform als Flexionsunterstützung kann die Vorrichtung grundsätzlich auch zur Extensionsunterstützung eingesetzt werden, die Ausführungen zur Flexionsunterstützung gelten entsprechend auch für eine Extensionsunterstützung, wobei es auch möglich und vorgesehen ist, dass eine Flexions- und Extensionsunterstützung gemeinsam in einer Gelenkeinrichtung angeordnet sind.

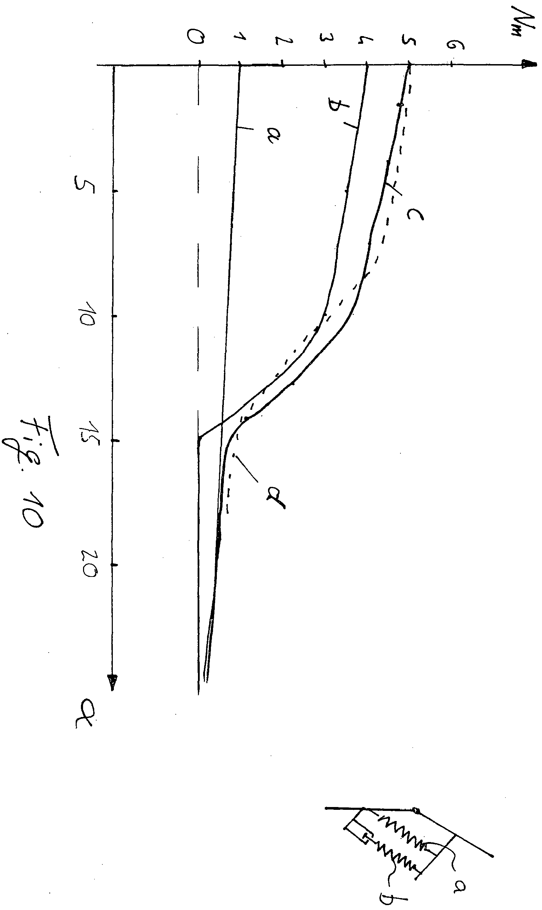

In der Figur 10 ist ein Momentenverlauf zweier parallel geschalteter Federn a, b dargestellt, wobei das Unterstützungsmoment in Nm über den Winkel α des Gelenkwinkels aufgetragen ist. Im dargestellten Ausführungsbeispiel handelt es sich um einen Momentenverlauf für ein Unterstützungsmoment bei der Schwungsphaseneinleitung eines orthetischen oder prothetischen Kniegelenks. Das notwendige Unterstützungsmoment in der Schwungphaseneinleitung ist nicht linear und in Gestalt einer Strichlinie mit dem Bezugszeichen d bezeichnet. Grundsätzlich ist es möglich, eine solche Federkennlinie oder ein solches Moment über eine lineare Feder zu erzeugen, wobei diese mit einem Dämpfer oder einem Aktor gekoppelt ist, um die lineare Feder in ihrem Verhalten zu beeinflussen. Statt eines Aktors oder eines Dämpfers ist in der Figur 10 die Zusammenschaltung zweier Federn mit unterschiedlichen Federsteifigkeiten gezeigt, die über verschiedene Winkelbereiche wirksam sind. Die erste Feder a weist einen nahezu linearen Momentenverlauf über die unterschiedlichen Winkel α auf. Die Federsteifigkeit ist geringer als bei der zweiten Feder b, die eine höhere Federsteifigkeit aufweist, jedoch nur bis zu einem Winkel von α = 15° wirksam ist. Die Kombination beider Federn a, b führt zu einem Momentenverlauf c, der dem gewünschten Momentenverlauf d sehr gut angenähert ist. Der Momentenverlauf der Feder b sieht vor, dass sie nur bis zu einem Beugewinkel von 15° wirksam ist, danach ist die gespeicherte Energie umgewandelt und trägt nicht mehr zur weiteren Kniebeugung bei. Dies kann beispielsweise dadurch geschehen, dass die Feder b als Zugfeder ausgeführt und in einer Längsführung geführt ist, wie in der oberen rechten Darstellung eines Gelenks mit parallel geschalteten Federn a, b dargestellt ist. Die erste Feder a ist über einen längeren Verschwenkweg oder Verschwenkwinkel α hinweg wirksam, während die Feder b, die ebenfalls als Zugfeder ausgebildet ist, so ausgebildet ist und in ihrer Länge so bemessen ist, dass sie nach Erreichen des vorbestimmten Winkels keinen Beitrag mehr zur weiteren Einbeugung des Gelenkes beiträgt. Die erste Feder a weist eine geringere Steifigkeit als die zweite Feder b auf und wirkt über einen längeren Zeitraum oder Winkelbereich, so dass sich beim Zusammenschalten beider Federn der kombinierte Momentenverlauf c ergibt, der dem gewünschten Momentenverlauf d sehr nahe kommt. Der gewünschte Verlauf d kann aus Experimenten ermittelt werden, insbesondere bei einem Prothesenkniegelenk oder Orthesenkniegelenk kann sich der Momentenverlauf für unterschiedliche Gehgeschwindigkeiten verändern, so dass bei einer angenommenen mittleren Gehgeschwindigkeit ein guter Kompromiss für alle Geschwindigkeiten gefunden werden kann.

Ein Vorteil der Anordnung ist es, dass die gesamte in den Federn a, b gespeicherte Energie für die Einbeugung des Gelenks verwendet werden kann und keine externe Energie für einen Aktor aufgewendet oder in den Feder a, b gespeicherte Energie über einen Dämpfer dissipiert werden muss. Bei nur einer Feder müsste diese stärker ausgelegt und überschüssige Energie in einem bestimmten Winkelbereich über einen Dämpfer umgewandelt werden.

## Patentansprüche

1. Verfahren zur Steuerung einer orthopädietechnischen Gelenkeinrichtung (1) einer unteren Extremität mit einem Oberteil (2) und einem gelenkig daran gelagerten Unterteil (3), zwischen denen eine Umwandlungseinrichtung (5) angeordnet ist, über die während einer Verschwenkung des Oberteils (2) relativ zu dem Unterteil (3) mechanische Arbeit aus der Relativbewegung umgewandelt und in zumindest einem Energiespeicher (54) gespeichert und der Gelenkeinrichtung (1) zeitversetzt wieder zugeführt wird, um die Relativbewegung zu unterstützen, wobei die gespeicherte Energie rückgewandelt und die Zufuhr mechanischer Arbeit während der Unterstützung der Relativbewegung kontrolliert erfolgt, wobei die Umwandlungseinrichtung (5) und/oder der Energiespeicher (54) eine Kombination zumindest zweier Federn aufweist, die über verschiedene Winkelbereiche der Gelenkeinrichtung wirksam sind, **dadurch gekennzeichnet, dass** der Zeitpunkt eines in Eingriff Tretens der Umwandlungseinrichtung (5) zur Veränderung der umzuwandelnden Energiemenge und/oder der zugeführten Energiemenge verstellt wird.

2. Verfahren nach Anspruch 1, **dadurch gekennzeichnet, dass** die Zufuhr mechanischer Arbeit dadurch verändert wird, dass dem Energiespeicher (54) extern Energie zugeführt oder entzogen wird.

3. Verfahren nach Anspruch 1 oder 2, **dadurch gekennzeichnet, dass** dem Energiespeicher (54) ein Aktuator (10) zugeordnet ist, über den der Energiespeicher (54) auf ein Mindestniveau befüllt wird, wenn die Relativbewegung dazu nicht ausreicht.

4. Verfahren nach einem der voranstehenden Ansprüche, **dadurch gekennzeichnet, dass** dem Energiespeicher (54) eine Freigabeeinrichtung (14) zugeordnet ist, über die die Energie aus dem Energiespeicher (54) freigegeben wird.

5. Verfahren nach einem der voranstehenden Ansprüche, **dadurch gekennzeichnet, dass** die mechanische Arbeit in Abhängigkeit von zumindest einem folgenden Kriterium oder einer Kombination mehrerer folgender Kriterien zugeführt wird:
- der Winkelstellung des Oberteils (2) zu dem Unterteil (3),
- der Position des Oberteils (2) und/oder des Unterteils (3) im Raum,
- einer Winkelgeschwindigkeit des Oberteils (2) und/oder des Unterteils (3),
- der Relativgeschwindigkeit zwischen Oberteil (2) und Unterteil (3),
- der Belastungssituation ,
- der Beschleunigung des Oberteils (2) und/oder des Unterteils (3).

6. Verfahren nach einem der voranstehenden Ansprüche, **dadurch gekennzeichnet, dass** die Energie in Abhängigkeit von zumindest einem folgenden Kriterium oder einer Kombination mehrerer folgender Kriterien zugeführt oder entzogen wird:
- der Winkelstellung des Oberteils (2) zu dem Unterteil (3),
- der Position des Oberteils (2) und/oder des Unterteils (3) im Raum,
- einer Winkelgeschwindigkeit des Oberteils (2) und/oder des Unterteils (3),
- der Relativgeschwindigkeit zwischen Oberteil (2) und Unterteil (3),
- der Belastungssituation ,
- der Beschleunigung des Oberteils (2) und/oder des Unterteils (3).

7. Verfahren nach einem der voranstehenden Ansprüche, **dadurch gekennzeichnet, dass** der Energiespeicher (54) von einem Aktuator (10) aufgeladen wird, wenn die Umwandlungseinrichtung (5) nicht aufgrund der Relativbewegung zwischen dem Oberteil (2) und dem Unterteil (3) aktiv ist.

8. Verfahren nach einem der voranstehenden Ansprüche, **dadurch gekennzeichnet, dass** die Relativbewegung über eine Dämpfereinrichtung (50) beeinflusst wird.

9. Orthopädietechnische Gelenkeinrichtung einer unteren Extremität mit einem Oberteil (2) und einem gelenkig daran gelagerten Unterteil (3), zwischen denen eine Umwandlungseinrichtung (5) angeordnet ist, über die während einer Verschwenkung des Oberteils (2) relativ zu dem Unterteil (3) mechanische Arbeit aus der Relativbewegung umgewandelt und in zumindest einem Energiespeicher (54) gespeichert und der Gelenkeinrichtung (1) zeitversetzt wieder zugeführt wird, um die Relativbewegung zu unterstützen, wobei dem Energiespeicher (54) ein Aktuator (10) zugeordnet ist, der während der Unterstützung der Relativbewegung dem Energiespeicher (54) kontrolliert Energie zuführt oder entzieht, wobei die Umwandlungseinrichtung (5) und/oder der Energiespeicher (54) eine Kombination zumindest zweier Federn aufweist, die über verschiedene Winkelbereiche der Gelenkeinrichtung wirksam sind, **dadurch gekennzeichnet, dass** die Umwandlungseinrichtung (5) verstellbar mit dem Oberteil (2) und/oder dem Unterteil (3) gekoppelt ist, um eine Eingriffsposition oder einen Verstellweg zu verlagern.

10. Orthopädietechnische Gelenkeinrichtung nach Anspruch 9, **dadurch gekennzeichnet, dass** die Umwandlungseinrichtung (5) als Feder, Federspeicher oder Wandler ausgebildet ist.

11. Orthopädietechnische Gelenkeinrichtung nach Anspruch 9 oder 10, **dadurch gekennzeichnet, dass** der Energiespeicher (54) als Feder oder Akkumulator ausgebildet ist.

12. Orthopädietechnische Gelenkeinrichtung nach einem der Ansprüche 9 bis 11, **dadurch gekennzeichnet, dass** eine separate Dämpfereinrichtung (50) zwischen dem Oberteil (2) und dem Unterteil (3) angeordnet ist.

13. Orthopädietechnische Gelenkeinrichtung nach Anspruch 12, **dadurch gekennzeichnet, dass** die separate Dämpfereinrichtung (50) verstellbar ausgebildet ist.

14. Orthopädietechnische Gelenkeinrichtung nach Anspruch 9, **dadurch gekennzeichnet, dass** die Federn unterschiedliche Federsteifigkeiten aufweisen.

## Claims

1. A method for controlling an orthopedic joint device (1) of a lower extremity with an upper part (2) and a lower part (3) mounted thereon in an articulated manner, between which a conversion device (5) is arranged, by means of which mechanical work from the relative movement during a pivoting of the upper part (2) relative to the lower part (3) is converted and stored in at least one energy store (54) and re-supplied to the joint device (1) in a time-offset manner in order to assist the relative movement, whereas the stored energy is reconverted and the supply of mechanical work when assisting the relative movement is carried out in a controlled manner, whereas the conversion device (5) and/or the energy store (54) has a combination of at least two springs, which are effective over different angular ranges of the joint device, **characterized in that** the time of engagement of the conversion device (5) is adjusted in order to modify the amount of energy to be converted and/or the amount of energy supplied.

2. The method as claimed in claim 1, **characterized in that** the supply of mechanical work is modified by virtue of energy being externally supplied to, or removed from, the energy store (54).

3. The method as claimed in claim 1 or 2, **characterized in that** an actuator (10) is assigned to the energy store (54), by means of which actuator the energy store (54) is filled to a minimum level if the relative movement is insufficient therefor.

4. The method as claimed in one of the preceding claims, **characterized in that** a release device (14) is assigned to the energy store (54), by means of which release device the energy is released from the energy store (54).

5. The method as claimed in one of the preceding claims, **characterized in that** the mechanical work is supplied in a manner dependent on at least one of the following criteria or a combination of a plurality of the following criteria:
- the angular position of the upper part (2) relative to the lower part (3),
- the position of the upper part (2) and/or the lower part (3) in space,
- an angular velocity of the upper part (2) and/or the lower part (3),
- the relative velocity between upper part (2) and lower part (3),
- the load situation,
- the acceleration of the upper part (2) and/or the lower part (3).

6. The method as claimed in one of the preceding claims, **characterized in that** the energy is supplied or removed in a manner dependent on at least one of the following criteria or a combination of a plurality of the following criteria:
- the angular position of the upper part (2) relative to the lower part (3),
- the position of the upper part (2) and/or the lower part (3) in space,
- an angular velocity of the upper part (2) and/or the lower part (3),
- the relative velocity between upper part (2) and lower part (3),
- the load situation,
- the acceleration of the upper part (2) and/or the lower part (3).

7. The method as claimed in one of the preceding claims, **characterized in that** the energy store (54) is charged by an actuator (10) if the conversion device (5) is not active due to the relative movement between the upper part (2) and the lower part (3).

8. The method as claimed in one of the preceding claims, **characterized in that** the relative movement is influenced by way of the damper device (50).

9. An orthopedic joint device of a lower extremity with an upper part (2) and a lower part (3) mounted thereon in an articulated manner, between which a conversion device (5) is arranged, by means of which mechanical work from the relative movement during a pivoting of the upper part (2) relative to the lower part (3) is converted and stored in at least one energy store (54) and re-supplied to the joint device (1) in a time-offset manner in order to assist the relative movement, whereas an actuator (10) is assigned to the energy store (54), which actuator supplies energy to, or removes energy from, the energy store (54) in a controlled manner when assisting the relative movement, whereas the conversion device (5) and/or the energy store (54) has a combination of at least two springs, which are effective over different angular ranges of the joint device, **characterized in that** the conversion device (5) is coupled in an adjustable manner to the upper part (2) and/or the lower part (3) in order to displace an engagement position or an adjustment travel.

10. The orthopedic joint device as claimed in claim 10, **characterized in that** the conversion device (5) is embodied as a spring, spring mechanism or transducer.

11. The orthopedic joint device as claimed in claim 9 or 10, **characterized in that** the energy store (54) is embodied as a spring or accumulator.

12. The orthopedic joint device as claimed in one of claims 9 to 11, **characterized in that** a separate damper device (50) is arranged between the upper part (2) and the lower part (3).

13. The orthopedic joint device as claimed in claim 12, **characterized in that** the separate damper device (50) has an adjustable embodiment.

14. The orthopedic joint device as claimed in claim 9, **characterized in that** the springs have different spring constants.

## Revendications

1. Procédé pour commander un dispositif formant articulation orthopédique (1) d'une extrémité inférieure, comportant une partie supérieure (2) et une partie inférieure (3) qui y est montée en articulation, entre lesquelles est agencé un moyen de conversion (5) par lequel, pendant un basculement de la partie supérieure (2) par rapport à la partie inférieure (3), un travail mécanique issu du mouvement relatif est converti et est stocké dans au moins un accumulateur d'énergie (54) et retourné de façon décalée dans le temps au dispositif formant articulation (1), afin de soutenir le mouvement relatif, l'énergie stockée est reconvertie, et l'apport de travail mécanique s'effectue de façon contrôlée pendant le soutien du mouvement relatif, le moyen de conversion (5) et/ou l'accumulateur d'énergie (54) comprenant une combinaison d'au moins deux ressorts qui agissent sur différentes zones angulaires du dispositif formant articulation,
**caractérisé en ce que**
l'instant d'un engagement du moyen de conversion (5) est réglé pour modifier la quantité d'énergie à convertir et/ou la quantité d'énergie apportée.

2. Procédé selon la revendication 1,
**caractérisé en ce que**
l'apport de travail mécanique est modifié du fait que de l'énergie est apportée ou soutirée de l'accumulateur d'énergie (54) par voie externe.

3. Procédé selon la revendication 1 ou 2,
**caractérisé en ce que**
un actionneur (10) est associé à l'accumulateur d'énergie (54), par lequel l'accumulateur d'énergie (54) est rempli à un niveau minimum lorsque le mouvement relatif n'y suffit pas.

4. Procédé selon l'une des revendications précédentes,
**caractérisé en ce que**
un moyen de libération (14) est associé à l'accumulateur d'énergie (54), par lequel l'énergie est libérée de l'accumulateur d'énergie (54).

5. Procédé selon l'une des revendications précédentes,
**caractérisé en ce que**
le travail mécanique est apporté en fonction de l'un au moins des critères suivants ou d'une combinaison de plusieurs des critères suivants :
- la position angulaire de la partie supérieure (2) par rapport à la partie inférieure (3),
- la position de la partie supérieure (2) et/ou de la partie inférieure (3) dans l'espace,
- une vitesse angulaire de la partie supérieure (2) et/ou de la partie inférieure (3),
- la vitesse relative entre la partie supérieure (2) et la partie inférieure (3),
- la situation de charge,
- l'accélération de la partie supérieure (2) et/ou de la partie inférieure (3).

6. Procédé selon l'une des revendications précédentes,
**caractérisé en ce que**
l'énergie est apportée ou soutirée en fonction de l'un au moins des critères suivants ou d'une combinaison de plusieurs des critères suivants :
- la position angulaire de la partie supérieure (2) par rapport à la partie inférieure (3),
- la position de la partie supérieure (2) et/ou de la partie inférieure (3) dans l'espace,
- une vitesse angulaire de la partie supérieure (2) et/ou de la partie inférieure (3),
- la vitesse relative entre la partie supérieure (2) et la partie inférieure (3),
- la situation de charge,
- l'accélération de la partie supérieure (2) et/ou de la partie inférieure (3).

7. Procédé selon l'une des revendications précédentes,
**caractérisé en ce que**
l'accumulateur d'énergie (54) est chargé par un actionneur (10) lorsque le moyen de conversion (5) n'est pas actif en raison du mouvement relatif entre la partie supérieure (2) et la partie inférieure (3).

8. Procédé selon l'une des revendications précédentes,
**caractérisé en ce que**
le mouvement relatif est influencé par un moyen d'amortissement (50).

9. Dispositif formant articulation orthopédique (1) d'une extrémité inférieure, comportant une partie supérieure (2) et une partie inférieure (3) qui y est montée en articulation, entre lesquelles est agencé un moyen de conversion (5) par lequel, pendant un basculement de la partie supérieure (2) par rapport à la partie inférieure (3), un travail mécanique issu du mouvement relatif est converti et est stocké dans au moins un accumulateur d'énergie (54) et est retourné de façon décalée dans le temps au dispositif formant articulation (1), afin de soutenir le mouvement relatif, un actionneur (10) étant associé à l'accumulateur d'énergie (54), qui apporte ou soutire de façon contrôlée l'énergie de l'accumulateur d'énergie (54), pendant le soutien du mouvement relatif, le moyen de conversion (5) et/ou l'accumulateur d'énergie (54) comprenant une combinaison d'au moins deux ressorts qui agissent sur différentes zones angulaires du dispositif formant articulation,
**caractérisé en ce que**
le moyen de conversion (5) est couplé à la partie supérieure (2) et/ou à la partie inférieure (3) de façon réglable afin de déplacer une position d'engagement ou un trajet de déplacement.

10. Dispositif formant articulation orthopédique selon la revendication 9,
**caractérisé en ce que**
le moyen de conversion (5) est réalisé sous forme de ressort, d'accumulateur à ressort ou de convertisseur.

11. Dispositif formant articulation orthopédique selon la revendication 9 ou 10,
**caractérisé en ce que**
l'accumulateur d'énergie (54) est réalisé sous forme de ressort ou d'accumulateur.

12. Dispositif formant articulation orthopédique selon l'une des revendications 9 à 11,
**caractérisé en ce que**
un moyen d'amortissement (50) séparé est agencé entre la partie supérieure (2) et la partie inférieure (3).

13. Dispositif formant articulation orthopédique selon la revendication 12,
**caractérisé en ce que**
le moyen d'amortissement (50) séparé est réalisé de façon réglable.

14. Dispositif formant articulation orthopédique selon la revendication 9,
**caractérisé en ce que**
les ressorts présentent différentes raideurs.
